# DEMANDE DE BREVET EUROPEEN

(11) **EP 3 760 182 A1**
(43) Date de publication de la demande: **06.01.2021**
(21) Numéro de dépôt: 20305749.2
(22) Date de dépôt: 03.07.2020
(51) Int. Cl.: A61K 8/02, A61K 8/36, A61K 8/73, A61Q 1/08, A61Q 1/12, A61K 8/31, A61K 8/92

(54) **COMPOSITION COSMÉTIQUE SOLIDE LONGUE TENUE**

(30) Priorité: 03.07.2019 FR 1907395
(71) Demandeur: Chanel Parfums Beauté, 92200 Neuilly-sur-Seine (FR)
(72) Inventeur: DUPUIS, Lina, 93694 PANTIN CEDEX (FR); GIBERT, Cyril, 93694 PANTIN CEDEX (FR)
(74) Mandataire: Plasseraud IP

(57) **Abrégé**

La présente invention a pour objet une composition cosmétique solide comprenant au moins 15 % en poids d'une phase grasse liquide volatile, 40 à 85% en poids d'une phase pulvérulente comprenant des charges, sphériques, traitées en surface par un savon métallique. L'invention se rapporte également à un procédé de préparation d'une telle composition cosmétique solide et à un procédé de maquillage de la peau ou des lèvres la mettant en oeuvre.

## Description

La présente invention a pour objet une composition cosmétique solide comprenant au moins 15 % en poids d'une phase grasse liquide volatile, 40 à 85% en poids d'une phase pulvérulente comprenant des charges sphériques traitées en surface par un savon métallique. L'invention se rapporte également à un procédé de préparation d'une telle composition cosmétique solide et à un procédé de maquillage de la peau ou des lèvres la mettant en œuvre.

### Domaine technique

Les formes galéniques classiquement retenues pour les compositions solides sont généralement des poudres libres ou compactes. A titre illustratif et non limitatif des formes galéniques solides plus particulièrement considérées dans le domaine du maquillage, on peut notamment citer poudres pour le teint, les fards à joues ou les fards à paupières.

Les poudres précitées ont principalement pour fonction d'apporter de la couleur, de la matité ou encore conférer de la couvrance. D'une manière générale, les poudres associent une phase pulvérulente largement majoritaire à une phase grasse au moins en partie liquide constituant le liant et permettant, dans le cadre des poudres compactes, d'assurer une bonne cohésion de la phase pulvérulente.

Une phase pulvérulente est formée pour l'essentiel de charges et d'agents de coloration, la quantité de ceux-ci étant modulée pour procurer l'effet de maquillage recherché, coloriel, couvrant ou matifiant. Lorsque le pourcentage de phase pulvérulente au sein du produit devient trop important, sa fabrication et son compactage deviennent compliqués voire impossible à réaliser à un niveau industriel compte tenu des exigences de qualité et de productivité. De plus, de telles compositions solides avec une teneur importante en phase pulvérulente peuvent présenter l'inconvénient d'être inconfortables, trop sèches et trop poudreuses et pour certaines d'être fragiles, cassantes, avec une mauvaise résistance aux chocs. Enfin, des quantités importantes de phase pulvérulente dans la poudre compacte ne donnent pas des propriétés sensorielles satisfaisantes, les rendant difficiles à déliter lorsqu'on prélève la poudre de son conditionnement (« pick-up ») et/ou à étaler lorsqu'on l'applique sur la surface de la peau à maquiller (« pay-off »).

Pour obtenir une composition sous forme solide, il est connu de mettre en œuvre un procédé de compactage (« dry process ») consistant à mélanger la phase pulvérulente et la phase grasse et à compacter sous haute pression la composition résultante dans un boîtier. Alternativement, un procédé qualifié de « wet process » (ou « slurry ») peut être mis en œuvre pour réaliser de telles compositions. Dans ce type de procédé, la phase pulvérulente et la phase grasse de ladite composition sont mises en présence d'un solvant volatil de manière à former une suspension, laquelle est ensuite pressée et le solvant volatil éliminé.

Quel que soit le procédé considéré, la quantité de phase grasse, et notamment d'huiles, n'excède généralement pas 10% de la composition de façon à obtenir un bon compactage de la poudre via les moyens mécaniques, et également éviter tout débordement de la composition du boîtier. Pour ces raisons, ces galéniques obligent bien souvent les formulateurs à limiter la quantité de phase grasse, et en particulier d'huile(s), de manière à assurer un bon compactage de la poudre.

Lorsqu'on augmente la quantité de liant gras, la mise en forme de la composition solide par compactage devient donc compliquée, voire impossible. Pour répondre à cette contrainte industrielle, des produits solides plus pâteux, par exemple obtenus par extrusion, ont été proposés. Toutefois, en présence d'une teneur trop importante en phase liante, la composition a tendance à cirer, c'est-à-dire à durcir lors de l'utilisation, et devient trop dense jusqu'à empêcher son prélèvement et altérer ses propriétés d'étalement à l'application.

En outre, les compositions solides sous forme de poudre ne présentent pas toujours une tenue dans le temps de la couvrance et de la couleur satisfaisante.

On demeure à la recherche de compositions cosmétiques solides présentant une bonne cohésion et une bonne résistance aux chocs, en particulier pouvant être stockées et transportées librement par l'utilisatrice sans s'effriter ni se fissurer, dont la texture permet un délitage et une application aisés, et présentant une bonne tenue dans le temps de la couvrance et de la couleur.

La demanderesse a découvert de façon inattendue qu'une composition présentant de telles propriétés, a priori non conciliables, pouvait être obtenue en mettant en œuvre, dans une composition solide, une teneur spécifique d'une phase grasse liquide particulière, et une phase pulvérulente au moins en partie traitée en surface par un savon métallique.

L'invention a ainsi pour objet, selon un premier aspect, une composition cosmétique solide comprenant :
- au moins 15 % en poids d'une phase grasse liquide volatile,
- 40 à 85% en poids d'une phase pulvérulente comprenant des charges sphériques traitées en surface par un savon métallique,
   les pourcentages étant exprimés en poids, par rapport au poids total de la composition.

En particulier, l'invention a pour objet une composition cosmétique solide comprenant :
- au moins 15% en poids d'une phase grasse liquide volatile,
- 40 à 85% en poids d'une phase pulvérulente comprenant des charges sphériques traités en surface par un savon métallique, et comprenant en outre une charge lamellaire traitée en surface par un savon métallique,
   les pourcentages étant exprimés en poids, par rapport au poids total de la composition.

En particulier, l'invention a pour objet une composition cosmétique solide comprenant :
- au moins 15% en poids d'une phase grasse liquide volatile,
- 40 à 85% en poids d'une phase pulvérulente comprenant des charges sphériques traitées en surface par un savon métallique, et comprenant en outre une nacre traitée en surface par un savon métallique,
   les pourcentages étant exprimés en poids, par rapport au poids total de la composition.

En particulier, l'invention a pour objet une composition cosmétique solide comprenant :
- au moins 15% en poids d'une phase grasse liquide volatile,
- 40 à 85% en poids d'une phase pulvérulente comprenant des charges sphériques traitées en surface par un savon métallique, et comprenant en outre une charge lamellaire traitée en surface par un savon métallique, et une nacre traitée en surface par un savon métallique,
   les pourcentages étant exprimés en poids, par rapport au poids total de la composition.

En particulier, l'invention a pour objet une composition cosmétique solide comprenant :
- 20 à 23% en poids d'une phase grasse liquide volatile, et
- 50 à 80% en poids de phase pulvérulente, de préférence de 60 à 75% en poids, les pourcentages étant exprimés en poids, par rapport au poids total de la composition.

L'invention a également pour objet, selon un deuxième aspect, un procédé de préparation d'une telle composition, comprenant :
- le pré-mélange des poudres constituant la phase pulvérulente
- la préparation d'un liant gras comprenant la phase grasse liquide volatile et le polymère filmogène
- l'empâtage des poudres avec le liant gras par extrusion, et
- la mise en forme de la composition par pressage.

L'invention a encore pour objet, selon un troisième aspect, un procédé de maquillage de la peau ou des lèvres, consistant à appliquer sur la peau ou les lèvres une telle composition cosmétique solide.

### Galénique

La composition selon l'invention est solide, en ce sens qu'elle ne s'écoule pas sous son propre poids. Elle se présente de préférence sous la forme d'un produit pâteux, obtenu par extrusion d'un mélange d'une phase pulvérulente et d'une phase grasse, optionnellement en présence d'un solvant volatil qui sera évaporé (procédé « slurry »).

### Phase grasse

La composition selon l'invention comprend au moins une phase grasse liquide volatile, c'est-à-dire une phase grasse comprenant au moins une huile volatile. Selon un mode de réalisation, elle peut en outre comprendre une phase grasse liquide non volatile, c'est-à-dire une phase grasse comprenant au moins une huile non volatile.

Par « huile non volatile », on entend une huile restant sur les fibres kératiniques à température ambiante et pression atmosphérique au moins plusieurs heures et ayant notamment une pression de vapeur inférieure à 10⁻³mm de Hg (0,13 Pa).

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les alcanes linéaires en C4 à C36, de préférence C11-C21 comme le phyto squalane ou l'Emogreen L15 de SEPPIC (alcane en C15-19), ou encore telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de C4 à C36 , et, notamment, de C18 à C36 ; ces huiles pouvant être linéaires ou ramifiées, saturées ou insaturées ; ces huiles peuvent, notamment, être des triglycérides héptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol (Helianthus Annuus seed oil), l'huile de ricin, et l'huile de watermelon, l'éthyl olivate comme le Vegeflow D10 d'Innovation company et leurs mélanges, ou encore des triglycérides d'acides caprylique/caprique, comme ceux vendus par la société STEARINERIES DUB0IS ou ceux vendus sous les dénominations MIGLYOL 810® , 812® et 818® par la société DYNAMIT NOBEL,
- les éthers de synthèse ayant de 10 à 40 atomes de carbone ;
- les esters de synthèse, comme les huiles de formule R1 COOR2, dans laquelle R1 représente un reste d'un acide gras linéaire ou ramifié comportant de 1 à 40 atomes de carbone et R2 représente une chaîne hydrocarbonée, notamment, ramifiée contenant de 1 à 40 atomes de carbone à condition que R1 + R2 soit ≥ 10. Les esters peuvent être, notamment, choisis parmi les esters d'alcool et d'acide gras, comme par exemple l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylène glycol et leurs mélanges, les benzoates d'alcools en C12-C15 , le laurate d'hexyle, les esters de l'acide néopentanoïque, comme le néopentanoate d'isodécyle, le néopentanoate d'isotridécyle, le néopentanoate d'isostéaryle, le néopentanoate d'octyldocécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyols et les esters du pentaérythritol, comme le tétrahydroxystéarate/tétraisostéarate de dipentaérythritol,
- les esters de dimères diols et de dimères diacides, tels que les Lusplan DD-DA5® et Lusplan DD-DA7® , commercialisés par la société NIPPON FINE CHEMICAL et décrits dans la demande US 2004-175338 ,
- les copolymères de dimère diol et de dimère diacide et leurs esters, tels que les copolymères dimères dilinoleyl diol/dimères dilinoléiques et leurs esters, comme par exemple le Plandool-G,
- les copolymères de polyols et de dimères diacides, et leurs esters, tels que le Hailuscent ISDA,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique, le 2-hexyldécanol, le 2-blatyloctanol, et le 2-undécylpentadécanol,
- les acides gras supérieurs en C₁₂-C₂₂ , tels que l'acide oléique, l'acide linoléique, et leurs mélanges,
- les carbonates de di-alkyle, les 2 chaînes alkyles pouvant être identiques ou différentes, tels que le dicaprylyl carbonate commercialisé sous la dénomination CETIOL CC®, par COGNIS,
- les huiles de masse molaire élevée ayant, en particulier, une masse molaire allant d'environ 400 à environ 10 000 g/mol, en particulier, d'environ 650 à environ 10 000 g/mol, en particulier, d'environ 750 à environ 7500 g/mol, et plus particulièrement, variant d'environ 1000 à environ 5000 g/mol,
- les huiles siliconées, telles que les silicones phénylées comme la BELSIL PDM 1000 de la société WACIER (MM=9000 g/mol) ou les huiles de silicone non phénylées telles que les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phénol triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges,
- les huiles fluorées utilisables dans l'invention sont notamment des huiles fluorosiliconées, des polyéthers fluorés, des silicones fluorées telles que décrit dans le document EP-A-847752.

Selon un mode particulier de réalisation, la composition selon l'invention comprend, en outre, une phase grasse liquide non volatile, comprenant au moins une huile non volatile.

Selon un mode particulier de réalisation, l'huile non volatile mise en œuvre dans la composition selon l'invention est choisie parmi les huiles hydrocarbonées, les huiles siliconées non phénylées et leurs mélanges.

En particulier, la phase grasse liquide non volatile comprend de 0,1 à 10% en poids d'huile non volatile, de préférence de 1 à 5% en poids.

La composition selon l'invention comprend au moins une phase grasse liquide volatile, c'est-à-dire une phase grasse comprenant au moins une huile volatile.

Par « huile volatile », on entend au sens de l'invention une huile susceptible de s'évaporer au contact des fibres kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

En particulier, la phase grasse liquide volatile peut comprendre au moins une huile volatile choisie parmi les huiles hydrocarbonées, les huiles siliconées et leurs mélanges.

L'huile volatile peut être hydrocarbonée. L'huile volatile hydrocarbonée peut être choisie parmi les huiles hydrocarbonées ayant de 7 à 16 atomes de carbone. Comme huile volatile hydrocarbonée ayant de 7 à 16 atomes de carbone, on peut citer notamment les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée ayant de 8 à 16 atomes de carbone est choisie parmi l'isododécane, l'isodécane, l'isohexadécane et leurs mélanges, et est notamment l'isododécane.

L'huile volatile peut être un alcane linéaire volatil. Selon un mode de réalisation, un alcane convenant à l'invention peut être un alcane linéaire volatil comprenant de 7 à 14 atomes de carbone. Un tel alcane linéaire volatil peut être avantageusement d'origine végétale. A titre d'exemple d'alcanes convenant à l'invention, on peut mentionner les alcanes décrits dans les demandes de brevets de la société Cognis WO 2007/1068371, ou WO2008/155059 (mélanges d'alcanes distincts et différant d'au moins un carbone). Ces alcanes sont obtenus partir d'alcools gras, eux-mêmes obtenus à partir d'huile de coprah ou de palme. A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges. Selon un mode préféré, on peut citer les mélanges de n-undécane (C11) et de n-tridécane (C13) obtenus aux exemples 1 et 2 de la demande WO2008/15505 de la Société Cognis. On pourra également citer le mélange de n-undécane (C11) et de n-tridécane (C13) commercialisé par la société BASF sous le nom de CETIOL ULTIMATE. On peut encose citer le n-dodécane (C12) et le n-tétradécane (C14) vendus par Sasol respectivement sous les références PARAFOL 12-97 et PARAFOL 14-97, ainsi que leurs mélanges. On peut encore citer le C9-C12 alkane commercialisé sous la référence VEGELIGHT SILK par la société Biosynthis. On pourra utiliser l'alcane linéaire volatil seul ou préférentiellement un mélange d'au moins deux alcanes linéaires volatils distincts, différant entre eux d'un nombre de carbone n d'au moins 1, en particulier différant entre eux d'un nombre de carbone de 1 ou de 2.

L'huile volatile peut être une huile siliconée volatile telle que les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane et le dodecamethylcyclohexasiloxane.

En variante ou de façon additionnelle, la composition réalisée peut comprendre au moins une huile volatile fluorée.

Selon un mode préféré de réalisation, la composition selon l'invention comprend au moins 15 % en poids d'une phase grasse liquide volatile, de préférence de 20 à 23% en poids d'une phase grasse liquide volatile.

La composition selon l'invention peut également comprendre une phase grasse solide, comprenant au moins une cire et/ou un corps gras pâteux et/ou un gélifiant lipophile.

Selon un mode préféré de réalisation, la composition selon l'invention comprend de 0,1 à 15% en poids, de préférence de 0,5 à 10% en poids, d'une phase grasse solide.

### Cires

La composition selon l'invention peut comprendre au moins une cire.

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 120 °C.

En particulier, les cires convenant à l'invention peuvent présenter un point de fusion supérieur à 45 °C environ, et en particulier supérieur à 55 °C. Le point de fusion de la cire peut être mesuré à l'aide d'un calorimètre à balayage différentiel (D.S.C.), par exemple le calorimètre vendu sous la dénomination DSC 30 par la société METLER.

Les cires susceptibles d'être utilisées dans les compositions selon l'invention sont choisies parmi les cires, solides, déformables ou non à température ambiante, d'origine animale, végétale, minérale ou de synthèse et leurs mélanges.

La cire peut également présenter une dureté allant de 0,05 MPa à 30 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

On peut notamment utiliser les cires hydrocarbonées comme la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; la cire d'abeille, la cire de jojoba, la cire de mimosa, la cire de tournesol, les cires de polyéthylène, les cires obtenues par la synthèse de Fisher-Tropsch et les copolymères cireux ainsi que leurs esters. Un mélange de cire de jojoba, cire de mimosa, cire de tournesol est par exemple commercialisée sous la référence ACTICIRE MP par la société GATTEFOSSE. En particulier, les cires hydrocarbonées peuvent être choisies parmi la cire de Carnauba, la cire d'abeille, la cire de jojoba, la cire de mimosa, la cire de tournesol, et leurs mélanges.

On peut aussi citer les cires obtenues par hydrogénation catalytique d'huiles animales ou végétales ayant des chaînes grasses, linéaires ou ramifiées, en C₈-C₃₂.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transestérification et hydrogénation d'huiles végétales, telles que l'huile de ricin ou d'olive, comme les cires vendues sous les dénominations de Phytowax ricin 16L64® et 22L73® et Phytowax Olive 18L57 par la société SOPHIM. De telles cires sont décrites dans la demande FR-A-2792190.

On peut aussi utiliser des cires siliconées qui peuvent être avantageusement des polysiloxanes substitués, de préférence à bas point de fusion. Ces cires de silicones sont connues ou peuvent être préparées selon les méthodes connues. Parmi les cires de silicones commerciales de ce type, on peut citer notament celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC), les alkyle- ou alcoxydiméthicones tels que les produits commerciaux suivants : Abilwax 2428. 2434 et 2440 (GOLDSCHMIDT), ou VP 1622 et VP 1621 (WACKER), ainsi que les (C20-C60) alkyldiméthicones, en particulier les (C30-C45) alkyldiméthicones comme la cire siliconée vendue sous la dénomination SF-1642 par la société GE-Bayer Silicones.

On peut également utiliser des cires hydrocarbonées modifiées par des groupements siliconés ou fluorés comme par exemple : siliconyl candelilla, siliconyl beeswax et Fluorobeeswax de Koster Keunen.

Les cires peuvent également être choisies parmi les cires fluorées.

Selon un mode de réalisation particulier, les compositions selon l'invention peuvent comprendre au moins une cire dite cire collante. Comme cire collante, on peut utiliser un (hydroxystéaryloxy)stéarate d'alkyle en C20-C40(le groupe alkyle comprenant de 20 à 40 atomes de carbone), seul ou en mélange, en particulier un 12-(12'-hydroxystéaryloxy)stéarate d'alkyle en C20-C40. Une telle cire est notamment vendue sous les dénominations « Kester Wax K 82 P®» et « Kester Wax K 80 P®» par la société KOSTER KEUNEN.

Selon un mode préféré de réalisation, les cires sont choisies parmi les cires hydrocarbonées, de préférence choisies parmi la cire de Carnauba, la cire d'abeille, la cire de jojoba, la cire de mimosa, la cire de tournesol, et leurs mélanges.

### Corps gras pâteux

La phase grasse solide peut également comprendre un corps gras pâteux, hydrocarboné, siliconé et/ou fluoré, ou un mélange de ceux-ci.

Par "corps gras pâteux" au sens de la présente invention, on entend un composé gras lipophile à changement d'état solide/liquide réversible présentant à l'état solide une organisation cristalline anisotrope, et comportant à la température de 23 °C une fraction liquide et une fraction solide.

En d'autres termes, la température de fusion commençante du corps gras pâteux peut être inférieure à 23 °C. La fraction liquide du corps gras pâteux mesurée à 23 °C peut représenter 9 à 97 % en poids du corps gras pâteux. Cette fraction liquide à 23 °C représente de préférence entre 15 et 85 %, de préférence encore entre 40 et 85 % en poids.

Au sens de l'invention, la température de fusion correspond à la température du pic le plus endothermique observé en analyse thermique (DSC) telle que décrite dans la norme ISO 11357-3 ; 1999. Le point de fusion d'un corps gras pâteux peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de corps gras pâteux disposé dans un creuset est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de corps gras pâteux en fonction de la température. Le point de fusion du corps gras pâteux est la valeur de la température correspondant au sommet du pic de la courbe représentant la variation de la différence de puissance absorbée en fonction de la température.

La fraction liquide en poids du corps gras pâteux à 23 °C est égale au rapport de l'enthalpie de fusion consommée à 23 °C sur l'enthalpie de fusion du corps gras pâteux. L'enthalpie de fusion du corps gras pâteux est l'enthalpie consommée par ce dernier pour passer de l'état solide à l'état liquide. Le corps gras pâteux est dit à l'état solide lorsque l'intégralité de sa masse est sous forme solide cristalline. Le corps gras pâteux est dit à l'état liquide lorsque l'intégralité de sa masse est sous forme liquide.

L'enthalpie de fusion du corps gras pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999.

L'enthalpie de fusion du corps gras pâteux est la quantité d'énergie nécessaire pour faire passer le corps gras pâteux de l'état solide à l'état liquide. Elle est exprimée en J/g.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

La fraction liquide du corps gras pâteux mesurée à 32 °C représente de préférence de 30 à 100 % en poids du corps gras pâteux, de préférence de 50 à 100 %, de préférence encore de 60 à 100 % en poids du corps gras pâteux. Lorsque la fraction liquide du corps gras pâteux mesurée à 32 °C est égale à 100 %, la température de la fin de la plage de fusion du corps gras pâteux est inférieure ou égale à 32 °C.

La fraction liquide du corps gras pâteux mesurée à 32 °C est égale au rapport de l'enthalpie de fusion consommée à 32 °C sur l'enthalpie de fusion du corps gras pâteux. L'enthalpie de fusion consommée à 32 °C est calculée de la même façon que l'enthalpie de fusion consommée à 23 °C.

Le corps gras pâteux est de préférence choisi parmi les corps gras synthétiques et les corps gras d'origine végétale. Un corps gras pâteux peut être obtenu par synthèse à partir de produits de départ d'origine végétale.

Le corps gras pâteux est avantageusement choisi parmi :
- la lanoline et ses dérivés,
- les éthers de polyol choisi parmi les éthers de pentaérythritol et de polyalkylène glycol,
- les éthers d'alcool gras et de sucre, et leurs mélanges. l'éther pentaérythritol et de polyéthylène glycol comportant 5 motifs oxyéthylénés (5 OE) (nom CTFA: PEG-5- Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société VEVY, mélange où les constituants se trouvent dans un rapport en poids 46/46/8: 46 % de PEG5 Pentaerythrityl Ether, 46 % de PPG-5 Pentaerythrityl Ether et 8 % d'huile de soja,
- les composés silicones polymères ou non,
- les composés fluorés polymères ou non,
- les polymères vinyliques, notamment les homopolymères et les copolymères d'oléfines, les homopolymères et copolymères de diènes hydrogénés,
- les polyéthers liposolubles résultant de la polyéthérification entre un ou plusieurs diols en C2-C100, de préférence en C2-050,
- les esters,
   et/ou leurs mélanges.

Le corps gras pâteux est de préférence un polymère, notamment hydrocarboné.

Parmi les polyéthers liposolubles, on préfère en particulier les copolymères d'éthylèneoxyde et/ou de propylène-oxyde avec des alkylènes-oxydes à longue chaîne en C6-C30, de préférence encore tels que le rapport pondéral de l'éthylène-oxyde et/ou de propylèneoxyde avec alkylènes-oxydes dans le copolymère est de 5:95 à 70:30. Dans cette famille, on citera notamment les copolymères tels que les alkylènes-oxydes à longue chaîne disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par AKZO NOBEL.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique, l'acide isostéarique et l'acide 12-hydroxystéarique, à l'image notamment de ceux commercialisé sous la marque Softisan649 par la société SASOL,
- le propionate d'arachidyle commercialisé sous la marque Waxenol 801 par ALZO,
- les esters de phytostérol,
- les triglycérides d'acides gras et leurs dérivés,
- les esters de pentaérythritol,
- les esters de dimère diol et dimère diacide, le cas échéant, estérifiés sur leur(s) fonction(s) alcool(s) ou acide(s) libre(s) par des radicaux acides ou alcools, notamment les esters dimer dilinoleate; de tels esters peuvent être notamment choisis parmi les esters de nomenclature INCI suivante : le bis-béhényl/isostéaryl/phytostéryl dimerdilinoléyle dimerdilinoléate (Plandool G), le phytostéryl isostéaryl dimerdilinoléate (Lusplan PI-DA, Lusplan PHY/IS-DA), le phytostéryl/isostéryl/cétyl/stéaryl/béhényl dimerdilinoléate (Plandool H ou Plandool S) et leurs mélanges,
- le beurre de mangue, tel que celui commercialisé sous la référence Lipex 203 par la société AARHUSKARLSHAMN,
- l'huile de soja hydrogénée, l'huile de coprah hydrogénée, l'huile de colza hydrogénée, les mélanges d'huiles végétales hydrogénées tels que le mélange d'huile végétale hydrogénée de soja, coprah, palme et colza, par exemple le mélange commercialisé sous la référence Akogel® par la société AARHUSKARLSHAMN (nom INCI Hydrogenated Vegetable Oil),
- le beurre de karité, en particulier celui dont le nom INCI est Butyrospermum Parkii Butter, tel que celui commercialisé sous la référence Sheasoft® par la société AARHUSKARLSHAMN,
- le beurre de cacao, en particulier celui qui est commercialisé sous la dénomination CT COCOA BUTTER DEODORIZED par la société DUTCH COCOA BV ou celui qui est commercialisé sous la dénomination BEURRE DE CACAO NCB HD703 758 par la société BARRY CALLEBAUT ;
- le beurre de shorea, en particulier celui qui est commercialisé sous la dénomination DUB SHOREA T par la société STEARINERIE DUBOIS ;
   et leurs mélanges.

### Gélifiants lipophiles

Outre les cires, la composition selon l'invention peut comprendre au moins un gélifiant lipophile par exemple constitué par les copolymères de styrène et d'oléfines telles que l'éthylène, le propylène et/ou le butylène, éventuellement associés à des solvants siliconés ou hydrocarbonés, tels que décrits en particulier dans la demande WO 98/38981 et dans le brevet US-6,309,629, ou les copolymères de styrène et de butadiène tels que ceux commercialisés sous la référence OleaoFLEX EG 200 par la société Applechem. Ils comprennent notamment les gélifiants à base de terpolymères séquencés disponibles auprès de la société PENRECO sous la dénomination commerciale VERSAGEL®. Un autre type de gélifiant lipophile est constitué des polyamides tels que ceux identifiés par le nom INCI polyamide-3 et en particulier les polymères SYLVACLEAR® AF 1900V et PA 1200V disponibles auprès de la société ARIZONA CHEMICAL ainsi que ceux identifiés par le nom INCI « Ethylenediamine/Hydrogenated Dimer Dilinoleate Copolymer Bis-Di-C14-18 Alkyl Amide » et disponibles par exemple sous la dénomination commerciale SYLVACLEAR® A200V ou SYLVACLEAR® A2614V auprès de la société ARIZONA CHEMICAL. Le gélifiant lipophile peut en variante être une bentone ou une hectorite modifiée hydrophobe. Le gélifiant des huiles peut également être un gélifiant polyuréthane, de préférence d'origine naturelle tel qu'un dérivé d'huile de ricin disponible par exemple sous la dénomination commerciale EstoGel® M par la société Polymerexpert.

### Phase pulvérulente

La composition selon l'invention comprend également au moins une phase pulvérulente comprenant des charges, sphériques traitées en surface par un savon métallique. La phase pulvérulente peut comprendre également une charge lamellaire traitée en surface par un savon métallique, une nacre traitée en surface par un savon métallique ou les deux. La phase pulvérulente peut, de préférence, également comprendre des pigments, optionnellement traités en surface par un savon métallique.

### Traitement de surface

La phase pulvérulente mise en œuvre dans les compositions selon l'invention est au moins en partie traitée en surface par un savon métallique.

En particulier, la phase pulvérulente comprend des charges, sphériques traitées en surface par un savon métallique.

En particulier, la phase pulvérulente comprend des charges sphériques traitées en surface par un savon métallique et comprend en outre une charge lamellaire traitée en surface par un savon métallique.

En particulier, la phase pulvérulente comprend des charges sphériques traitées en surface par un savon métallique et comprend en outre une nacre traitée en surface par un savon métallique.

En particulier, la phase pulvérulente comprend des charges sphériques traitées en surface par un savon métallique et comprend en outre une charge lamellaire traitée en surface par un savon métallique et une nacre traitée en surface par un savon métallique.

En particulier, le savon métallique est un savon d'acides gras ayant de 12 à 22 atomes de carbone, et en particulier de 12 à 18 atomes de carbone.

Le métal du savon métallique est quant à lui de préférence choisi parmi le zinc et le magnésium.

Ainsi, selon un mode préféré de réalisation, le savon métallique est choisi parmi le laurate de zinc, le stéarate de magnésium, le myristate de magnésium, le stéarate de zinc, et leurs mélanges, et de préférence, le savon métallique est du stéarate de magnésium.

### Charges

Les charges peuvent être minérales ou organiques.

La phase pulvérulente comprend au moins une charge sphérique. Les charges sphériques sont avantageusement choisies parmi :
- les poudres de silice ;
- les poudres de (co)polymères acryliques, et leurs dérivés, en particulier les poudres de (co)polymère acrylate, et leurs dérivés, avantageusement choisies parmi une poudre de polyméthacrylate de méthyle, une poudre de polyméthacrylate de méthyle/diméthacrylate d'éthylène glycol, une poudre de polyméthacrylate d'allyle/diméthacrylate d'éthylène glycol, une poudre de copolymère diméthacrylate d'éthylène glycol/méthacrylate de lauryle, une poudre de copolymère acrylate / alkyl acrylate éventuellement réticulé, les particules creuses de (co-) polymère d'acrylonitrile expansées, et leur(s) mélange(s) ;
- les poudres de polyuréthane ;
- les poudres de silicone avantageusement choisies parmi une poudre de polyméthylsilsesquioxane, d'élastomère d'organopolysiloxane enrobée de résine de silicone, une poudre de particules organosiliconées ;
- les poudres de polyamide, tel que de Nylon®, en particulier Nylon 12 ;
- les poudres de cellulose, telles que les Cellulobeads D5, D10, D50 et D100 commercialisées par la société Daito,
   et leur(s) mélange(s).

La composition peut comprendre en outre une charge lamellaire. Parmi les charges lamellaires, on peut citer le talc, le mica naturel ou synthétique, certaines silices, les argiles tels que les silicate de magnésium et d'aluminium, le siloxysilicate de triméthyle, le kaolin, la bentone, le carbonate de calcium et l'hydrogéno-carbonate de magnésium, l'hydroxyapatite, le nitrure de bore, la fluorphlogopite, des poudres de perlite, une poudre N-Lauroyl Lysine, la séricite, le calcium sodium borosilicate, le calcium aluminium borosilicate, et leur(s) mélange(s).

Parmi les charges lamellaires, on préfère le talc, le mica naturel ou synthétique, certaines silices, les argiles tels que les silicate de magnésium et d'aluminium, le siloxysilicate de triméthyle, le kaolin, la bentone, le carbonate de calcium et l'hydrogéno-carbonate de magnésium, l'hydroxyapatite, la fluorphlogopite, des poudres de perlite, une poudre N-Lauroyl Lysine, la séricite, le calcium sodium borosilicate, le calcium aluminium borosilicate, et leur(s) mélange(s).

La composition peut comprendre en outre une charge additionnelle. La charge additionnelle peut être choisie parmi les charges minérales ou organiques de toute forme, lamellaires, sphériques (ou hémisphériques), quelle que soit la forme cristallographique (par exemple feuillet, cubique, hexagonale, orthorombique, etc).

Selon un mode préféré de réalisation, la phase pulvérulente comprend des charges lamellaires et sphériques en un ratio lamellaires/sphériques allant de 1/10 à 10/1, de préférence de 1/5 à 9/1. Ce ratio est un ratio pondéral.

Par « pigments », il faut comprendre des particules blanches ou colorées, minérales ou organiques, insolubles dans un milieu aqueux, destinées à colorer et/ou opacifier la composition.

Les pigments peuvent être blancs ou colorés, minéraux et/ou organiques.

Le pigment peut être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références C1 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI11725, 15510,45370, 71105,les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque. Par laque, on entend les colorants insolubilisés adsorbés sur des particules insolubles, l'ensemble ainsi obtenu restant insoluble lors de l'utilisation. A titre d'exemples de laques, on peut citer le produit connu sous la dénomination suivante : D & C Red 7 (CI 15 850:1).

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : Ti₂O₅, Ti₃O₅, Ti₂O₃, TiO, ZrO₂ en mélange avec TiCO₂, ZrO₂, Nb₂O₅, CeO₂, ZnS.

La taille du pigment utile dans le cadre de la présente invention est généralement comprise entre 10 nm et 10 µm, de préférence entre 20 nm et 5 µm, et plus préférentiellement entre 30 nm et 1 µm.

L'agent de coloration peut également être un colorant soluble, de préférence soluble dans l'eau.

Parmi les colorants solubles dans l'eau, on peut citer le carmin de cochenille ou les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985). D & C Green (CI 61 570), D & C Yellow 1 O (CI 77 002), D & C Green 3 (CI 42 053), D & C Blue 1 (CI 42 090).

Les nacres peuvent être choisies parmi celles classiquement présentes dans les produits de maquillage, telles que les mica / dioxyde de titane. En variante, il peut s'agir de nacres à base de mica / silice / dioxyde de titane, à base de fluorphlogopite synthétique / dioxyde de titane (SUNSHINE® de MAPRECOS), de calcium sodium borosilicate / dioxyde de titane (REFLECKS® d'ENGELHARD) ou de calcium aluminium borosilicate / silice / dioxyde de titane (RONASTAR® de MERCK).

La composition selon l'invention comprend de 40 à 85% en poids de phase pulvérulente, de préférence de 50 à 80% en poids, par rapport au poids total de la composition selon l'invention.

### Polyols

La composition selon l'invention peut également comprendre au moins un polyol.

On entend par polyol toute molécule organique présentant dans sa structure au moins 2 groupements hydroxy (-OH) libres. Ces polyols sont de préférence liquides à température ambiante (25°C).

A titre d'exemple de polyols convenant à la mise en œuvre dans la composition peuvent être choisis parmi le propylène glycol, le butylène glycol, le pentylène glycol, le pentanediol, l'isoprène glycol, le néopentyl glycol, le glycérol, les polyéthylène glycols (PEG) ayant notamment de 4 à 8 motifs éthylène glycol et/ou le sorbitol.

De préférence, le polyol est le glycérol.

Selon un mode particulier de réalisation, la composition selon l'invention comprend de 2 à 30% en poids de polyols, de préférence de 5 à 25% en poids, par rapport au poids total de la composition.

### Agent émulsionnant

La composition selon l'invention peut également comprendre un agent émulsionnant.

Ces agents émulsionnant peuvent être choisis parmi des tensioactifs non ioniques, anioniques, cationiques, amphotères ou encore des tensioactifs polymériques.

Selon un mode de réalisation, les tensioactifs pouvant être utilisés dans le cadre de l'invention sont choisis parmi les tensioactifs non ioniques de HLB compris entre 8 et 20 à 25 °C. On peut citer notamment :
- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8 -C24 , et de préférence en C12 -C18) tels que l'éther oxyéthyléné de l'alcool cétéarylique à 30 groupes oxyéthylénés (nom CTFA « Ceteareth-30 »), l'éther oxyéthyléné de l'alcool stéarylique à 20 groupes oxyéthylénés (nom CTFA « Stéareth-20 »), l'éther oxyéthyléné du mélange d'alcools gras en C12- C15 comportant 7 groupes oxyéthylénés (nom CTFA « C12-15 Pareth-7 ») notamment commercialisé sous la dénomination NEODOL 25-7® par SHELL CHEMICALS
- les esters d'acide gras (notamment d'acide en C8 -C24 , et de préférence en C16 -C22) et de polyéthylène glycol (pouvant comprendre de 1 à 150 motifs d'éthylèneglycol) tels que le stéarate de PEG-50 et le monostéarate de PEG-40 notamment, commercialisé sous le nom MYRJ 52P® par la société ICI UNIQUEMA, ou encore le PEG-30 glyceryl stéarate notamment commercialisé sous le nom TAGAT S® par la société Evonik GOLDSCHMIDT ;
- les esters d'acide gras (notamment d'acide en C8 -C24 , et de préférence en C16 -C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle notamment vendu sous la dénomination Simulsol 220 TM® par la société SEPPIC ; le stéarate de glycéryle polyéthoxylé à 30 groupes d'oxyde d'éthylène comme le produit TAGAT S® vendu par la société Evonik GOLDSCHMIDT, l'oléate de glycéryle polyéthoxylé à 30 groupes d'oxyde d'éthylène comme le produit TAGAT O® vendu par la société Evonik GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société Evonik GOLDSCHMIDT et le laurate de glycéryle polyéthoxylé à 30 groupes d'oxyde d'éthylène comme le produit TAGAT I® de la société Evonik GOLDSCHMIDT,
- les esters d'acide gras (notamment d'acide en C8 -C24 , et de préférence en C16 -C22) et des éthers de sorbitol oxyéthylénés et/ou oxypropylénés (pouvant comporter de 1 à 150 groupes oxyéthylénés et/ou oxypropylénés), comme le polysorbate 20 notamment vendu sous la dénomination Tween 20® par la société CRODA, le polysorbate 60 notamment vendu sous la dénomination Tween 60® par la société CRODA,

- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde de propylène et d'oxyde d'éthylène, également appelés polycondensats OE/OP
- les lysophospholipides , en particulier la lysophosphatidylcholine de formule [CHEM1] suivante:

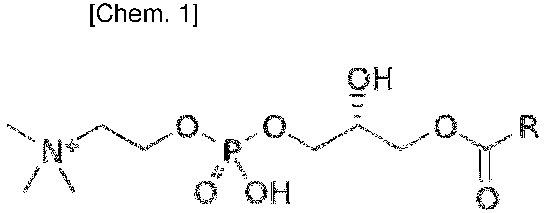

où R est une chaîne d'acide gras, comprenant notamment de 10 à 25 atomes de carbone, de préférence de 15 à 20. De préférence, le lysophospholipide utilisé dans la composition de l'invention est issu de graines de soja. De préférence encore, il a pour nom INCI glycine soja (soybean) seed extract. Par exemple, on utilise le mélange de glycérine à 80% en poids et de glycine soja (soybean) seed extract à 20% en poids commercialisé par Kemin sous la dénomination Lysofix Liquid® ; - les cires émulsionnantes telles que la cire autoémulsionnante vendue sous le nom de Polawax NF par Croda, ou la cire d'abeille PEG-8 vendue sous le nom d'Apifil par Gattefossé,
et leurs mélanges.

Selon un mode préféré de réalisation, l'agent émulsionnant de HLB compris entre 8 et 20 est choisi parmi les esters d'acide gras et des éthers de sorbitol oxyéthylénés et/ou oxypropylénés, et leurs mélanges.

Les lysophospholipides tels que le Lysofix Liquid® permettent un épaississement de la composition.

Selon un mode de réalisation, les tensioactifs pouvant être utilisés dans la composition selon l'invention sont choisis parmi les tensioactifs non ioniques de HLB inférieur ou égal à 8 à 25 °C. On peut citer notamment :
- les esters et éthers d'oses tels que le stéarate de sucrose, le cocoate de sucrose, le stéarate de sorbitan et leurs mélanges comme l'Arlatone 2121® commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en C8 -C24 , et de préférence en C12 -C18) tels que l'éther oxyéthyléné de l'alcool stéarylique à 2 groupes oxyéthylénés (nom CTFA « Stéareth-2 ») ;
- les esters d'acides gras (notamment d'acide en C8 -C24 , et de préférence en C16 C22) et de polyol, notamment de glycérol ou de sorbitol, tels que le stéarate de glycéryle, tel que le produit vendu sous la dénomination TEGIN M® par la société Evonik GOLDSCHMIDT, le laurate de glycéryle tel que le produit vendu sous la dénomination IMWITOR 312® par la société HULS, le stéarate de polyglycéryl-2, le triisostéarate de polyglycéryl-2, le tristéarate de sorbitan, le ricinoléate de glycéryle ;
- les lécithines, telles que les lécithines de soja (comme Emulmetik 100 J de Cargill, ou Biophilic H de Lucas Meyer) ;
- les émulsionnants siliconés.

L'émulsionnant siliconé pouvant être utilisé dans la composition selon l'invention est un polymère de siloxane comprenant :
- une chaîne latérale grasse,
- une chaîne latérale oxyéthylénée ou oxypropylénée et/ou une chaîne latérale polyéthoxylée (= glycérylée), et éventuellement
- une chaîne latérale siliconée.

La chaîne latérale grasse de l'émulsionnant siliconé permet d'avoir une bonne comptabilité avec la phase grasse de l'émulsion eau-dans-huile. La chaîne latérale siliconée permet d'avoir une bonne comptabilité avec l'huile siliconée non volatile, lorsque l'huile non volatile de l'émulsion cosmétique de l'invention est une huile siliconée. Selon un mode de réalisation de l'invention, l'émulsionnant siliconé comprend une chaîne latérale grasse et une chaîne latérale siliconée.

Plus particulièrement selon l'invention, l'émulsionnant siliconé est choisi dans le groupe comprenant :
- le composé de formule [CHEM2] suivante dans laquelle w est un entier allant de 1 à 1000, x' est un entier allant de 1 à 50, x, y et z représentent indépendamment les uns des autres un entier allant de 1 à 100: 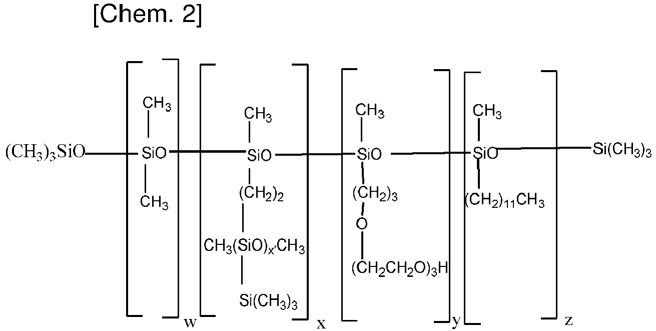
- le composé de formule [CHEM3] suivante dans laquelle x1 est un entier allant de 1 à 1000, w1 est un entier allant de 1 à 50, y1 et z1 représentent indépendamment l'un de l'autre un entier allant de 1 à 100 : 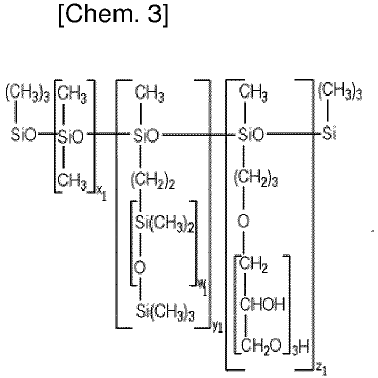
- le composé de formule [CHEM4] suivante dans laquelle w2 est un entier allant de 1 à 1000, v2 est un entier allant de 1 à 50, x2, y2 et z2 représentent indépendamment les uns des autres un entier allant de 1 à 100 : 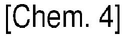 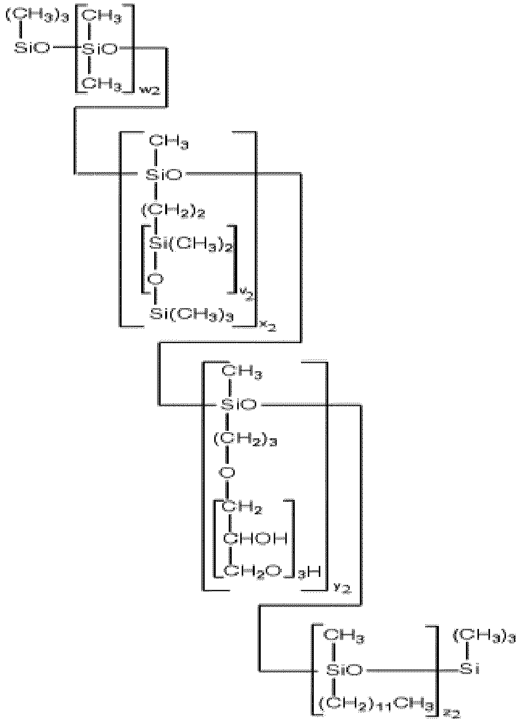 le mélange de cyclométhicone/diméthicone copolyol vendu sous la dénomination Q2-3225C® par la société DOW CORNING,
   et leurs mélanges.

Selon un mode particulier de réalisation, l'émulsionnant siliconé est choisi dans le groupe comprenant les polymères de siloxane commercialisés par la société SHIN-ETSU sous les références KF6038, KF6104, KF6105, KF6106 et leurs mélanges.

Le composé KF6038, ayant pour nom INCI « Lauryl PEG-9 Polydimethylsiloxyethyl Dimethicone » répond à la formule générale (I). Ce polymère de siloxane comprend une chaîne latérale siliconée, une chaîne latérale oxyéthylénée et une chaîne latérale grasse (lauryl).

Le composé KF6104, ayant pour nom INCI « Polyglyceryl-3 Polydimethylsiloxyethyl Dimethicone », et le composé KF6106, ayant pour nom INCI « Polyglyceryl-3 Polydimethylsiloxyethyl Dimethicone » répondent à la formule générale (II). Ce polymère de siloxane comprend une chaîne latérale siliconée et une chaîne latérale glycérylée.

Le composé KF6105, ayant pour nom INCI « Lauryl Polyglyceryl-3 Polydimethylsiloxyethyl Dimethicone » répond à la formule générale (III). Ce polymère de siloxane comprend une chaîne latérale siliconée, une chaîne latérale glycérylée et une chaîne latérale grasse (lauryl).

Dans une mode de réalisation préféré, en particulier lorsque la composition comprend des huiles siliconées, le tensioactif est choisi parmi les tensioactifs siliconés tel que le composé KF6028 ou le composé KF6038 ou leur mélange.

L'émulsionnant siliconé est présent dans la composition cosmétique de l'invention en une teneur allant de 0,1% à 5%, de préférence de 1% à 3%, les pourcentages étant des pourcentages en poids par rapport au poids total de la composition.

Dans une mode de réalisation préféré, en particulier lorsque la composition comprend des huiles hydrocarbonées, le tensioactif est choisi parmi les tensioactifs non-siliconés, de préférence le polysorbate 20.

La composition selon l'invention peut contenir de 0,1 à 5 % en poids d'agent émulsionnant, par rapport au poids total de ladite composition, de préférence de 1 à 3 % en poids.

### Polymère filmogène

La composition selon l'invention peut également comprendre au moins un polymère filmogène.

Parmi les polymères filmogènes utilisables dans les compositions de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique, l'acide itaconique et l'acide crotonique, et plus préférentiellement l'acide itaconique (par exemple un sel métallique de poly(acide itaconique) tel que celui commercialisé sous la référence commerciale REVCARE NE 100S par la société Itaconix).

Les esters de monomères acides sont avantageusement choisis parmi les esters de l'acide (méth)acrylique (encore appelé les (méth)acrylates), notamment des (méth)acrylates d'alkyle, en particulier d'alkyle en C1-C30, de préférence en C1-C20, des (méth)acrylates d'aryle, en particulier d'aryle en C6-C10, des (méth)acrylates d'hydroxyalkyle, en particulier d'hydroxyalkyle en C2-C6.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

On peut également citer les copolymères blocs styrène/butadiène tels que les produits de la société Kraton, ou l'OLEOFLEX EG 200 de la société APPLECHEM.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amine alcools. Comme diamine, on peut utiliser l'éthylènediamine, l'hexaméthylènediamine, la méta- ou para-phénylènediamine. Comme aminoalcool, on peut utiliser la monoéthanolamine. Comme résines polyamides, on peut également citer celle répondant à la dénomination INCI DIISOSTEARYL MALATE & BIS DIOCTADECYLAMIDE DIMER DILINOLEIC ACID/ETHYLENE DIAMINE COPOLYMER commercialisée sous l'appellation d'Haimalate PAM par la société Kokyu alcohol Kogyo.

Le polyester peut en outre comprendre au moins un monomère portant au moins un groupement -SO3M, avec M représentant un atome d'hydrogène, un ion ammonium NH4+ ou un ion métallique, comme par exemple un ion Na+, Li+, K+, Mg2+, Ca2+, Cu2+, Fe2+, Fe3+. On peut utiliser notamment un monomère aromatique bifonctionnel comportant un tel groupement -SO3M.

Le noyau aromatique du monomère aromatique bifonctionnel portant en outre un groupement -SO3M tel que décrit ci-dessus peut être choisi par exemple parmi les noyaux benzène, naphtalène, anthracène, diphényl, oxydiphényl, sulfonyldiphényl. méthylènediphényl. On peut citer comme exemple de monomère aromatique bifonctionnel portant en outre un groupement -SO3M : l'acide sulfoisophtalique, l'acide sulfotéréphtalique, l'acide sulfophtalique, l'acide 4-sulfonaphtalène-2,7-dicarboxylique.

On peut utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiées, peuvent être choisis parmi la résine shellac, la gomme de sandaraque, la gomme arabique (ACACIA SENEGAL GUM), les dammars, les élémis, les copals, les polymères cellulosiques, les polymères extraits du fruit de *Caesalpinia spinosa* et/ou de l'algue *Kappaphycus alvarezii* (tel que le produit Filmexel® commercialisé par la société Silab), et leurs mélanges. Un polymère naturel tel que le Filmexel® permet notamment d'améliorer la tenue du film obtenu à partie de la composition selon l'invention. On peut également citer les polymères filmogènes répondant au nom INCI SHOERA ROBUSTA RESIN + BEESWAX, SHOERA ROBUSTA RESIN + SUNFLOWER OIL, ARAUCARIA + SUNFLOWER OIL, ARAUCARIA + CASTOR OIL, SHOERA ROBUSTA + OCTYLDODECANOL

Selon un mode de réalisation, le polymère filmogène peut être un polymère solubilisé dans une phase grasse liquide comprenant des huiles ou solvants organiques (on dit alors que le polymère filmogène est un polymère liposoluble).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyle éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux allyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de méthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200000.

On peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly(méth)acrylates de lauryle, ces poly(méth)acrylates pouvant être réticulés à l'aide de diméthacrylate de l'éthylène glycol ou de tétraéthylène glycol.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrrolidone (VP) et notamment les copolymères de la vinylpyrrolidone et d'alcène en C2à C40et mieux en C3à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène (ANTARON V220 commercialisés par la société Ashland), VP/hexadécene (ANTARON V216 commercialisé par la société Ashland), VP/triacontène, VP/styrène, VP/acide acrylique/méthacrylate de lauryle.

On peut également citer les esters de dextrin et en particulier :
- le dextrin isostearate & isostearic acid commercialisé sous le nom UNIFILMA HVY par la société Chiba Flour Milling
- le dextrin palmitate /ethylhexanoate commercialisé sous le nom RHEOPEARL TT par la société Chiba Flour Milling
- le Dextrin Myristate commercialisé sous le nom RHEOPEARL MKL2 par la société Chiba Flour Milling

On peut encore citer les esters de sucre et en particulier le sucrose acetate isobutyrate commercialisé sous le nom de EASTMAN SUSTANE SAIB par la société EASTMAN.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomériques siloxane qu'elle comprend, chacune des lettres « MDTQ » caractérisant un type d'unité.

A titre d'exemples de résines polyméthylsilsesquioxanes commercialement disponibles, on peut citer celles qui sont commercialisés par la société Wacker sous la référence Resin MK tels que la Belsil PMS MK, et par la société SHIN-ETSU sous les références KR-220L, ou la silform fleible resin.

Comme résines siloxysilicates, on peut citer les résines triméthylsiloxysilicate (TMS) telles que celles commercialisées sous la référence SR1000 par la société General Electric ou sous la référence TMS 803 par la société Wacker. On peut encore citer les résines triméthylsiloxysilicate commercialisées dans un solvant tel que la cyclomethicone, vendues sous la dénomination « KF-7312J » par la société Shin-Etsu, «DOWSIL™ RSN-0749», «DOWSIL™ 593 Fluid» par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5,162,410 ou encore les copolymères siliconés issus de la réaction d'un résine de silicone, telle que celles décrite plus haut, et d'un diorganosiloxane tels que décrits dans le document WO 2004/073626.

On peut également utiliser les copolymères à squelette organique non-siliconé greffé par des monomères contenant un motif polysiloxane, comme par exemple le butyl acrylate /hydroxypropyl dimethicone acrylate copolymer commercialisé sous le nom de GRANACRYSIL BAS par la société GRANT.

On peut enfin citer les copolymères acrylate/polytriméthylsiloxyméthacrylate comprend une structure carbosiloxane dendrimère greffée sur un squelette vinylique disponible dans le commerce sous les références DOW CORNING FA 4002 ID ou DOW CORNING FA 4001 CM.

On peut également utiliser les polyamides siliconés du type polyorganosiloxane tels que ceux décrits dans les documents US-A-5.,874,069 , US-A-5,919,441 , US-A-6,051,216 et US-A-5,981,680 .

Dans un mode préféré de réalisation, la composition selon l'invention comprend de 1 à 15% en poids d'un polymère filmogène, de préférence 5 à 12% en poids d'au moins un polymère filmogène.

### Elastomères de silicone

La composition selon l'invention peut également comprendre un élastomère de silicone.

Parmi ceux-ci, on peut citer les polymères au moins partiellement réticulés résultant de la réaction d'un organopolysiloxane portant des groupes insaturés, tels que des groupes vinyle ou allyle, situés en bout ou en milieu de chaîne, de préférence sur un atome de silicium, avec un autre composé siliconé réactif tel qu'un organohydrogénopolysiloxane. Ces polymères sont habituellement disponibles sous forme de gel dans un solvant siliconé volatil ou non volatil ou dans un solvant hydrocarboné. Des exemples de tels élastomères sont notamment commercialisés par la société SHIN ETSU sous les dénominations commerciales KSG-6, KSG-16, KSG-31, KSG-32, KSG-41, KSG-42, KSG-43 et KSG-44, et par la société DOW CORNING sous les dénominations commerciales DOWSIL™ 9040 et DOWSIL™ 9041. Un autre gélifiant huileux est constitué d'un polymère de silicone, obtenu par auto-polymérisation d'un organopolysiloxane fonctionnalisé par des groupements epoxy et hydrosilylé, en présence d'un catalyseur, qui est disponible dans le commerce auprès de la société GENERAL ELECTRIC sous la dénomination commerciale VELVESIL® 125. Un autre gélifiant lipophile est constitué d'un copolymère dimethicone / vinyldimethicone cyclique tel que celui commercialisé par la société JEEN sous la dénomination commerciale JEESILC® PS (dont PS-VH, PS-VHLV, PS-CM, PS-CMLV et PS-DM).

Selon un mode préféré de réalisation, l'élastomère de silicone peut être émulsionnant, de préférence choisi parmi les elastomères siliconés polyoxyalkylénés et polyglycérolés.

Comme elastomères de silicone polyoxyalkylénés, on peut citer ceux décrits dans les brevets US5236986, US5412004, US5837793, US5811487.

Comme elastomères de silicone polyoxyalkylénés, on peut utiliser : ceux de nom INCI PEG-10 Dimethicone/Vinyl dimethicone crosspolymer : comme ceux commercialisés sous les dénominations "KSG-21 ", "KSG-20", par Shin Etsu ; - ceux de nom INCI Lauryl PEG- 15 Dimethicone/Vinyldimethicone Crosspolymer : comme ceux commercialisés sous les dénominations "KSG-30" et "KSG-31 ", KSG-32" (dans l'isododécane), "KSG-33" (dans la trioctanoine), "KSG-210", "KSG-310" (dans une huile minérale), "KSG-320" (dans l'isododécane), "KSG-330", "KSG-340" par la société Shin Etsu.

Comme élastomères de silicone polyglycérolés, on peut utiliser : - ceux de nom INCI Dimethicone (and) Dimethicone/Polyglycerin-3 crosspolymer : comme ceux commercialisés sous les dénominations "KSG-710" par Shin Etsu ; ceux de nom INCI Lauryl Dimethicone/Polyglycerin-3 crosspolymer: comme ceux commercialisés sous les dénominations "KSG-840" (dans du squalène) par la société Shin Etsu.

### Phase aqueuse

La composition selon l'invention peut également comprendre une phase aqueuse comprenant de l'eau et optionnellement, au moins un solvant soluble dans l'eau autre que les polyols précédemment décrits.

Par « solvant soluble dans l'eau », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent être volatils.

Parmi les solvants hydrosolubles pouvant être utilisés dans les compositions conformes à l'invention, on peut citer notamment les mono-alcools ayant de 1 à 5 atomes de carbone tels que l'éthanol et l'isopropanol, les cétones en C₃-C₄ et les aldéhydes en C₂-C₄.

Selon un mode préféré de réalisation, la composition selon l'invention est exempte d'eau.

### Actif cosmétique

La composition selon l'invention peut également comprendre au moins un actif cosmétique, qui peuvent être choisis dans le groupe constitué des vitamines, des antioxydants, des agents hydratants, des agents anti-pollution, les agents kératolytiques, des astringents, des anti-inflammatoires, des agents blanchissants, des auto-bronzants et des agents favorisant la microcirculation.

Des exemples de vitamines incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d'ascorbyle, l'ascorbyl glucoside, le magnésium ascorbyl phosphate, le sodium ascorbyl phosphate et le sorbate d'ascorbyle; le tocophérol et ses dérivés, tels que l'acétate de tocophérol, le sorbate de tocophérol et d'autres esters de tocophérol; le BHT et BHA; les esters de l'acide gallique, l'acide phosphorique, l'acide citrique, l'acide maléique, l'acide malonique, l'acide succinique, l'acide fumarique, la céphaline, l'hexamétaphosphate, l'acide phytique, et les extraits de plantes, par exemple de racines de Zingiber Officinale (Gingembre) tel que le Blue Malagasy Ginger commercialisé par la société BIOLANDES, de Chondrus crispus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le butylène glycol, le xylitol, le sorbitol, le maltitol, les mucopolysaccharides, tels que l'acide chondroïtine sulfurique, l'acide hyaluronique de haut ou de bas poids moléculaire ou encore l'acide hyaluronique potentialisé par un dérivé de silanol tel que l'actif Epidermosil® commercialisé par la société Exymol, et l'acide mucoitinsulfurique; l'acide caronique; l'atelo collagène; le chlorestéryl-12-hydroxystéarate; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; des dérivés de glycérine tels que le PEG / PPG / polybutylène Glycol-8/5/3 Glycérine de NOF vendu sous la dénomination commerciale Wilbride®S753 ou encore le glyceryl-polymethacrylate de Sederma vendu sous la dénomination commerciale Lubragel®MS; la triméthylglycine vendu sous la dénomination commerciale Aminocoat® par la société Ashahi Kasei Chemicals et divers extraits de plantes tels que des extraits de Castanea sativa, des protéines de noisette hydrolysées, les polysaccharides de Tuberosa Polyanthes, l'huile de noyau d'Argania spinosa et les extraits de nacre contenant un conchyoline qui sont vendus notamment par la compagnie Maruzen (Japon) sous le nom commercial Pearl Extract®.

D'autres exemples d'agents hydratants incluent les composés stimulant l'expression de la matriptase MT/SP1, tel qu'un extrait de pulpe de caroube, ainsi que les agents stimulant l'expression de CERT, d'ARNT2 ou de FN3K ou FN3K RP ; les agents augmentant la prolifération ou la différenciation des kératinocytes, soit directement, soit indirectement en stimulant par exemple la production de β-endorphines, tels que les extraits de *Thermus thermophilus* ou de coques de fèves de *Theobroma cacao*, les extraits hydrosolubles de maïs, les extraits peptidiques de *Voandzeia subterranea* et le niacinamide ; les lipides épidermiques et les agents augmentant la synthèse de lipides épidermiques, soit directement, soit en stimulant certaines β-glucosidases qui modulent la déglycosylation de précurseurs lipidiques comme le glucosylcéramide en céramides, tels que les phospholipides, les céramides, les hydrolysats de protéine de lupin et les dérivés d'acide dihydrojasmonique.

Des exemples d'agents anti-pollution incluent l'extrait de graines de Moringa pterygosperma (par exemple le Purisoft® de LSN); l'extrait de beurre de karité (par exemple Detoxyl® de Silab), un mélange d'extrait de lierre, d'acide phytique, d'extrait de graine de tournesol (par exemple l'Osmopur® de Sederma).

Des exemples d'agents kératolytiques incluent les α-hydroxyacides (par exemple les acides glycolique, lactique, citrique, malique, mandélique, ou tartrique) et les β-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'astringents incluent les extraits d'hamamélis.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Des exemples d'agents blanchissants incluent l'arbutine et ses dérivés, l'acide férulique (tel que le Cytovector® : eau, glycol, lécithine, acide férulique, hydroxyéthylcellulose, commercialisé par BASF) et ses dérivés, l'acide kojique, le résorcinol, l'acide lipoïque et ses dérivés tel que le monolipoate de resvératrol diacétate tel que décrit dans la demande de brevet WO2006134282, l'acide ellagique, le leucodopachrome et ses dérivés, la vitamine B3, l'acide linoléique et ses dérivés, les céramides et leurs homologues, un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique, un extrait de réglisse (extrait de Glycyrrhiza glabra), qui est vendu notamment par la société Maruzen sous le nom commercial Licorice extract®, un agent blanchissant ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et le sodium ascorbyl phosphate, ou les esters de saccharide d'acide ascorbique, qui incluent, par exemple, l'ascorbyle-2-glucoside, le L-ascorbate de 2-O-alpha-D-glucopyranosyle, ou le L-ascorbate de 6-O-bêta-D-galactopyranosyle. Un agent actif de ce type est vendu en particulier par la société DKSH sous le nom commercial Ascorbyl glucoside®.

Un exemple d'autobronzant est la DHA.

Des exemples d'agents favorisant la microcirculation incluent un extrait de lupin (tel que l'Eclaline® de Silab), de ruscus, de marron d'inde, de lierre, de ginseng ou de mélilot, la caféine, le nicotinate et ses dérivés, un extrait d'algue de Corallina officinalis tel que celui commercialisé par CODIF ; et leurs mélanges. Ces agents actifs sur la microcirculation cutanée peuvent être utilisés pour éviter le ternissement du teint et/ou améliorer l'homogénéisation et l'éclat du teint.

### Additifs

La composition selon l'invention peut comprendre d'autres ingrédients pour autant qu'ils n'interfèrent pas avec les propriétés souhaitées de la composition. Ces autres ingrédients peuvent par exemple être des conservateurs, des ajusteurs de pH tels l'acide citrique ou l'arginine, des agents antimicrobiens, des parfums, des filtres solaires, et leurs mélanges.

### Procédé de préparation

La présente invention a également pour objet un procédé de préparation d'une composition cosmétique solide selon l'invention, comprenant :
- le pré-mélange des poudres constituant la
- la préparation d'un liant gras comprenant la phase grasse liquide volatile et le polymère filmogène
- l'empâtage des poudres avec le liant gras par extrusion, et
- la mise en forme de la composition par pressage.

### Procédé de maquillage de matières kératiniques

La présente invention concerne également un procédé de maquillage de la peau ou des lèvres, consistant à appliquer sur la peau ou les lèvres une composition cosmétique solide selon l'invention.

### Exemple

### Ombres à paupières

On a préparé des ombres à paupières solides ayant la composition présentée dans le tableau 1 suivant.

**[Tableau 1]**

| Nom INCI | Teneur (% en poids) | | | |
|---|---|---|---|---|
| | OAP 1 | OAP 2 | | |
| pigments inorganiques (non enrobés) | | | | |
| pigments inorganiques enrobés stéarate de magnésium | | | | |
| pigments organiques | | | | |
| Nacres (non enrobées) | 8,63 | 8,63 | | |
| Nacres enrobées stéarate de magnésium | 41,18 | 41,18 | | |
| Charges lamellaires enrobées stéarate de magnésium | | | | |
| Charges sphériques (Cellulobeads) enrobées stéarate de magnésium | 14,60 | 14,60 | | |
| Trimethylsiloxysilicate (Belsil TMS803) | 5,40 | 5,40 | | |
| Butyl acrylate / hydroxypropyl dimethicone acrylate copolymer (Granacrysil BAS) | 3,65 | 3,65 | | |
| Dimethicone crosspolymer issu du DOWSIL 9041 (% DOWSIL 9041) | 0,22 (1,46) | 0,22 (1,46)) | | |
| Dimethicone issue du DOWSIL 9041 | 1,24 | 1,24 | | |
| Ethylhexyl polyhydrosystearate (Dub Estoline) | 1,46 | 1,46 | | |
| PEG-9 polydimethylsiloxyethyldimethicone (KF6028) | 0,73 | 0,73 | | |
| Isododécane | 21,60 | | | |
| C9-12 alcane (Vegelight Silk) | | 21,60 | | |

On a pré-mélangé les poudres constituant la phase pulvérulente.
On a ensuite préparé le liant gras non volatile comprenant la phase grasse (liquide et solide).
On a empâté les poudres avec le liant gras non volatile par extrusion puis on a empâté avec le solvant volatile additionnel par extrusion en introduisant le solvant volatile dans un orifice en aval du dispositif d'extrusion.
On a ensuite mis en forme les compositions par pressage.

Les ombres à paupières obtenues présentent une bonne cohésion et une bonne résistance aux chocs. En particulier, elles peuvent être stockées et transportées sans s'effriter ni se fissurer. Leur texture permet un délitage et une application aisés. Les ombres à paupières présentent une bonne tenue dans le temps de la couvrance et de la couleur.

## Revendications

1. Composition cosmétique solide comprenant:
- au moins 15 % en poids d'une phase grasse liquide volatile, et
- 40 à 85% en poids d'une phase pulvérulente comprenant des charges, sphériques, traitées en surface par un savon métallique, et
les pourcentages étant exprimés en poids, par rapport au poids total de la composition.

2. Composition cosmétique solide selon la revendication 1, **caractérisée en ce que** la phase pulvérulente comprend en outre une charge lamellaire traitée en surface par un savon métallique.

3. Composition cosmétique solide selon l'une quelconque des revendications 1 à 2, **caractérisée en ce que** la phase pulvérulente comprend en outre une nacre traitée en surface par un savon métallique.

4. Composition cosmétique solide selon l'une quelconque des revendications 1 à 3, **caractérisée en ce qu'**elle comprend :
- 20 à 23% en poids d'une phase grasse liquide volatile, et
- 50 à 80% en poids de phase pulvérulente, de préférence de 60 à 75% en poids.

5. Composition cosmétique solide selon l'une quelconque des revendications précédentes, **caractérisée en ce qu'**elle comprend 1 à 15% en poids d'un polymère filmogène, de préférence 5 à 12% en poids.

6. Composition cosmétique solide selon l'une quelconque des revendications précédentes, **caractérisée en ce que** la phase pulvérulente comprend des pigments, optionnellement traités en surface par un savon métallique.

7. Composition cosmétique solide selon l'une quelconque des revendications précédentes, **caractérisée en ce que** le savon métallique est un savon d'acides gras ayant de 12 à 22 atomes de carbone, et en particulier de 12 à 18 atomes de carbone.

8. Composition cosmétique solide selon l'une quelconque des revendications précédentes, **caractérisée en ce que** le métal du savon métallique est du zinc ou du magnésium.

9. Composition cosmétique solide selon l'une quelconque des revendications précédentes, **caractérisée en ce que** le savon métallique est choisi parmi le laurate de zinc, le stéarate de magnésium, le myristate de magnésium, le stéarate de zinc, et leurs mélanges, et de préférence, le savon métallique est du stéarate de magnésium.

10. Composition cosmétique solide selon l'une quelconque des revendications précédentes, **caractérisée en ce que** la phase grasse liquide volatile comprend au moins une huile volatile choisie parmi les huiles hydrocarbonées, les huiles siliconées et leurs mélanges.

11. Composition cosmétique solide selon l'une quelconque des revendications précédentes, **caractérisée en ce qu'**elle comprend une phase grasse liquide non volatile.

12. Composition cosmétique solide selon l'une quelconque des revendications précédentes, **caractérisée en ce qu'**elle comprend un agent émulsionnant.

13. Composition cosmétique solide selon l'une quelconque des revendications précédentes, **caractérisée en ce qu'**elle comprend une phase grasse solide.

14. Composition cosmétique solide selon la revendication 11 **caractérisée en ce qu'**elle comprend de 0,1 à 10% en poids d'huile non volatile, de préférence de 1 à 5% en poids.

15. Composition cosmétique solide selon l'une quelconque des revendications précédentes, **caractérisée en ce qu'**elle est exempte d'eau.

16. Composition cosmétique solide selon l'une quelconque des revendications précédentes, **caractérisée en ce qu'**elle comprend un élastomère de silicone.

17. Procédé de préparation d'une composition cosmétique solide selon l'une quelconque des revendications précédentes, comprenant :
- le pré-mélange des poudres constituant la phase pulvérulente
- la préparation d'un liant gras comprenant la phase grasse liquide volatile et le polymère filmogène
- l'empâtage des poudres avec le liant gras par extrusion, et
- la mise en forme de la composition par pressage.

18. Procédé de maquillage de la peau ou des lèvres, consistant à appliquer sur la peau ou les lèvres une composition cosmétique solide selon l'une quelconque des revendications 1 à 16.
